# FASCICULE DE BREVET EUROPEEN

(11) **EP 4 051 249 B1**
(45) Date de publication et mention de la délivrance du brevet: **13.09.2023**
(21) Numéro de dépôt: 20786346.5
(22) Date de dépôt: 01.10.2020
(51) Int. Cl.: A61K 31/03, A61K 9/107, A61K 9/16, A61P 35/00, A61K 9/14

(54) **COMPOSITION PHARMACEUTIQUE COMPRENANT LE MITOTANE POUR UNE ADMINISTRATION PAR VOIE ORALE POUR LE TRAITEMENT DU CARCINOME CORTICOSURRENALIEN ET DU SYNDROME DE CUSHING**
PHARMAZEUTISCHE ZUBEREITUNG ENTHALTEND MITOTANE ZUR ORALE VERABREICHUNG ZUR ANWENDUNG IN DER BEHANDLUNG VON NEBENNIERENRINDENKARZINOM UND CUSHING-SYNDROM
PHARMACEUTICAL COMPOSITION COMPRISING MITOTANE FOR ORAL ADMINISTRATION FOR USE IN THE TREATMENT OF ADRENOCORTICAL CARCINOMA AND CUSHING-SYNDROME

(30) Priorité: 28.10.2019 FR 1912084
(43) Date de publication de la demande: 07.09.2022
(73) Titulaire: Skiba, Mohamed, 76520 Montmain (FR)
(72) Inventeur: LAHIANI-SKIBA, Malika, 76520 Montmain (FR); BOUNOURE, Frédéric, 76230 Bois Guillaume (FR); THOMAS, Michael, 76130 Mont-Saint-Aignan (FR); LEFEBVRE, Hervé, 76960 N-D de Bondeville (FR); SKIBA, Mohamed, 76520 Montmain (FR)
(74) Mandataire: Danubia Patent & Law Office LLC
(86) Numéro de dépôt international: PCT/IB2020/059218
(87) Numéro de publication internationale: WO 2021/084345

(56) Documents cités:
- US-A- 4 146 648
- US-A- 5 472 706
- ALFONSI R ET AL: "Characterization of mitotane (o,p '-DDD) - cyclodextrin inclusion complexes: Phase-solubility method and NMR", ANNALES PHARMACEUTIQUES FRANCAISES, vol. 71, no. 3, mai 2013 (2013-05), pages 186-192, XP002799404, ISSN: 0003-4509
- ATTIVI D ET AL: "FORMULATION ET PHARMACOCINÉTIQUE DE SYSTÈMES AUTOÉMULSIONNANTS DE MITOTANE (O,P'-DDD)", INTERNET CITATION, 15 juin 2008 (2008-06-15), page 1, XP002545683, Extrait de l'Internet: URL:http://www.snphpu.com/infos.asp?InNum= In00001562&ThNum=Th0000005 [extrait le 2008-07-07]
- DATABASE BIOSIS [Online] BIOSCIENCES INFORMATION SERVICE, PHILADELPHIA, PA, US; May 2013 (2013-05), ALFONSI R ET AL: "Characterization of mitotane (o,p '-DDD) - cyclodextrin inclusion complexes: Phase-solubility method and NMR", Database accession no. PREV201300478220 & ANNALES PHARMACEUTIQUES FRANCAISES, vol. 71, no. 3, May 2013 (2013-05), pages 186-192, ISSN: 0003-4509, DOI: 10.1016/J.PHARMA.2013.02.001

## Description

Le corticosurrénalome ou carcinome corticosurrénalien est une tumeur cancéreuse rare qui se développe au niveau du cortex surrénalien (Else T, et al,. Endocr Rev 2014 35, 282-326 ; Fassnacht M, et al,. Nat Rev Endocrinol 2011 7,323-335). On estime l'incidence annuelle de 0,7 à 2 nouveaux cas par million d'habitants par an (Fassnacht M et al, J Clin Endocrinol Metab, 2013, 98 : 4551-4564), responsables de 0,04 à 0,2 % des décès pour cause de cancer.

Le carcinome corticosurrénalien survient le plus souvent chez l'adulte entre 40 et 50 ans mais également chez l'enfant de moins de 15 ans. Cette tumeur est plus souvent observée chez la femme que chez l'homme, sans qu'on en connaisse la raison mais le pronostic de cette maladie est sombre car elle est diagnostiquée tardivement et son traitement médical est très peu efficace avec une survie à 5 ans inférieure dans 40% des cas (Assié G et al, J Clin Endocrinol Metab, 2007, 92 : 148-154). L'exérèse chirurgicale totale de la tumeur représente la meilleure chance de guérison totale. Un traitement adjuvant peut-être proposé en complément à la chirurgie. Celui-ci consiste en l'administration par voie orale d'un dérivé de l'insecticide DDT, o',p'-DDD (ortho,para'dichloro-diphényl-dichloroéthane ou mitotane) le seul approuve pour le carcinome corticosurrénalien de formule chimique.

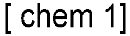

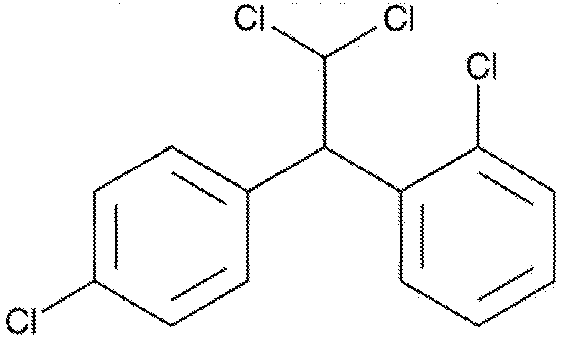

### Structure chimique du mitotane

De même, dans les formes avancées non opérables de la pathologie, seul le mitotane est proposé car il reste à ce jour la seule molécule avec une efficacité cytotoxique partielle pour le traitement des corticosurrénalomes. Une spécialité pharmaceutique (Lysodren^{®}) fait l'objet d'une AMM européenne depuis 2004 et d'une AMM aux USA depuis 1970 après la première étude clinque réalisée par l'équipe de R. H. Moy en 1960(D.M. Bergenstal et al., Chemotherapy of adrenocortical cancer Vol 53,4, 1960), avec pour indications officielles « le traitement du carcinome surrénalien dans les formes avancées chez le patient inopérable, dans les formes métastatiques, ou dans les formes récidivantes » et comme traitement palliatif en cas de maladie avancée.

La maladie de Cushing est causée par une tumeur hypophysaire sécrétant de l'hormone corticotrope (ACTH) et est la cause la plus fréquente de sécrétion excessive de cortisol endogène (Lacroix A, et al., Lancet. 2015;386(9996):913-927. doi: 10.1016/S0140-6736(14)61375-1 ; Biller BMK, et al., J Clin Endocrinol Metab. 2008;93(7):2454-2462. doi: 10.1210/jc.2007-2734. ; Pivonello R et al., Endocr Rev. 2015;36(4):385-486. doi: 10.1210/er.2013-1048). L'hypercortisolisme peut entraîner une morbidité importante et un décès prématuré par rapport à la population générale (Pivonello R et al., Endocrinol Metab Clin N Am. 2008;37(1):135-149. doi: 10.1016/j.ecl.2007.10.010). Les principaux objectifs de traitement de la maladie de Cushing sont de normaliser les niveaux de cortisol et de renverser les signes et les symptômes de l'hypercortisolisme (Biller BMK, et al., J Clin Endocrinol Metab. 2008;93(7) :2454-2462. doi: 10.1210/jc.2007-2734.; Pivonello R et al., Endocr Rev. 2015;36(4) :385-486. doi: 10.1210/er.2013-1048). Le traitement de première intention est la chirurgie transsphénoïdale (Biller BMK, et al., J Clin Endocrinol Metab. 2008;93(7):2454-2462. doi: 10.1210/jc.2007-2734), bien que cela ne réussisse pas toujours (Tritos NA et al., Nat Rev Endocrinol. 2011;7(5) :279-289. doi: 10.1038/nrendo.2011.12) et que les patients puissent rechuter plusieurs années après un succès chirurgical apparent(Dimopoulou C, et al., Eur J Endocrinol. 2013;170(2):283-292. doi: 10.1530/EJE-13-0634).

Un certain nombre de thérapies médicales sont actuellement utilisées en pratique clinique pour le traitement de la maladie de Cushing. Celles - ci comprennent le mitotane (agent adrénolytique), pasiréotide (analogue de la somatostatine), cabergoline (agoniste des récepteurs de la dopamine), la métyrapone et le kétoconazole (inhibiteurs de la stéroïdogenèse surrénale) et mifepristone (antagoniste du récepteur de glucocorticoïde). Étant donné que tous les patients atteints de la maladie de Cushing ne tirent pas un bénéfice suffisant des traitements disponibles, de nouvelles formulations sont toujours nécessaires.

Lorsque le Mitotane est administré sous la forme d'un comprimé conventionnel chez l'homme, sa biodisponibilité orale est médiocre avec une valeur de 35 à 40% après 3 mois d'administration, c'est-à-dire que 60% du produit se retrouve dans les fèces sous une forme non métabolisée du fait de sa très mauvaise solubilité aqueuse (solubilité : 1,29.10⁻⁷ mol/l à 25°C) (Hahner S & Fassnacht M.,Curr. Opin.Investig.Drugs 2005 6 386-394 ; Igaz P.et al, Med Chem 2008 15 2734-2747) .

Cette molécule active, pour être efficace, doit atteindre une concentration plasmatique d'au moins **14 mg/L** (Terzolo M, et, al., J Clin Endocrinol Metab 2000 85 2234-2238; Terzolo M, et, al., Curr Opin Endocrinol Diabètes Obes 2014 21 159-165). Celle-ci ne sera atteinte qu'au bout de **3 mois** en moyenne de traitement sans bénéfice thérapeutique pour le patient au cours de cette période. Ce temps de latence est dû au moins en partie au fait que le mitotane s'accumule préférentiellement dans les graisses à des concentrations qui peuvent représenter **200 fois** celle du plasma, diminuant ainsi sa biodisponibilité et son efficacité thérapeutique. Cette accumulation dans les graisses, associée à une faible biodisponibilité, oblige à augmenter les doses administrées au patient à des niveaux importants **(10 à 15 comprimés par jour).** Des effets secondaires sévères ont ainsi été identifiés dont les plus fréquents sont les troubles digestifs (nausées, vomissements et diarrhées) directement liés à la mauvaise absorption de la molécule et des troubles neurologiques (ataxie, syndrome dépressif...) lorsque la mitotanemie dépasse 20 mg/L. Récemment, des réactions lichenoides orales et vulvo-vaginales et des encéphalopathies ont été reportées également (Schmouchkovitch A.et al., Medecine (Baltimore) 2017 ;96 (2) : e5057 ; Betty Y. Lung et al., J. Clin oncol 33,2015 (suppl : abst 4105) ; E. Pare et al., the oncologiste 2017, 22 :1-2).

Le recours à des formulations lipidiques est une stratégie pour reformuler le mitotane, comme les systèmes autoémulsionnants (SAE) qui sont composés de tensioactifs, de co-solvants et d'huiles. Ce ne sont pas eux-mêmes des émulsions, mais sous agitation douce dans le milieu aqueux de l'estomac, ils forment des émulsions de taille submicronique facilement stables. Ces formes galéniques présentent des propriétés particulières. Elles peuvent modifier la composition du contenu gastrointestinal, interagir avec des transporteurs membranaires et/ou stimuler le transport des principes actifs par la voie lymphatique. L'absorption de ces formulations, après une administration par voie orale, peut se faire par le biais du système lymphatique qui permet d'éviter l'effet du premier passage hépatique. Le débit lymphatique étant plus lent que le débit sanguin, cela peut prolonger l'absorption du principe actif *(*Singh et al., Crit Rev Ther Drug Carrier Syst,2009, 26, 427-521) .

Par conséquent, les SAE à base de mitotane offrent la possibilité d'améliorer la biodisponibilité orale du mitotane, permettent de réduire le temps de traitement avec un bénéfice thérapeutique et de limiter le nombre de comprimés administrés par jour.

Plusieurs produits pharmaceutiques de ce type sont ainsi apparus sur le marché, basés sur cette stratégie de formulation avec l'administration de doses moyennes comme avec les spécialités Neoral^{®} (cyclosporine A) et Kaletra^{®} (lopinavir et le ritonavir), ou de doses plus faibles comme avec les spécialités Rocaltrol^{®} (Calcitriol) et Avodart^{®} (Dutasteride). Mais, jusqu'à présent, les SAE sont commercialisés uniquement sous forme de capsules molles, qui, bien que simples à réaliser, présentent plusieurs inconvénients. Comme le coût élevé de fabrication en raison du faible taux de production, le piégeage de l'air dans la capsule à haute vitesse de remplissage et l'incompatibilité éventuelle des composants du SAE avec l'enveloppe de la capsule pouvant réduire la durée de vie du produit (Cole, ET; et al., Adv. Drug Deliv. Rev. 2008, 60, 747 à 756) .

Trois tentatives ont été faites pour résoudre les problèmes liés à la formulation des systèmes autoémulsionnants (SAE)à base de mitotane.
- Un premier système autoémulsionnant (SAE) de mitotane a été développé par Attivi et collaborateurs (Attivi et al, Drug Dev Ind Pharm. 2010 Apr; 36(4) :421-7), comprenant du mitotane dissous dans une matrice composée d'un mélange égal de capryol^{®} 90, Tween^{®}20 et Cremophor EL^{®} (1/3 : 1/3 : 1/3) .

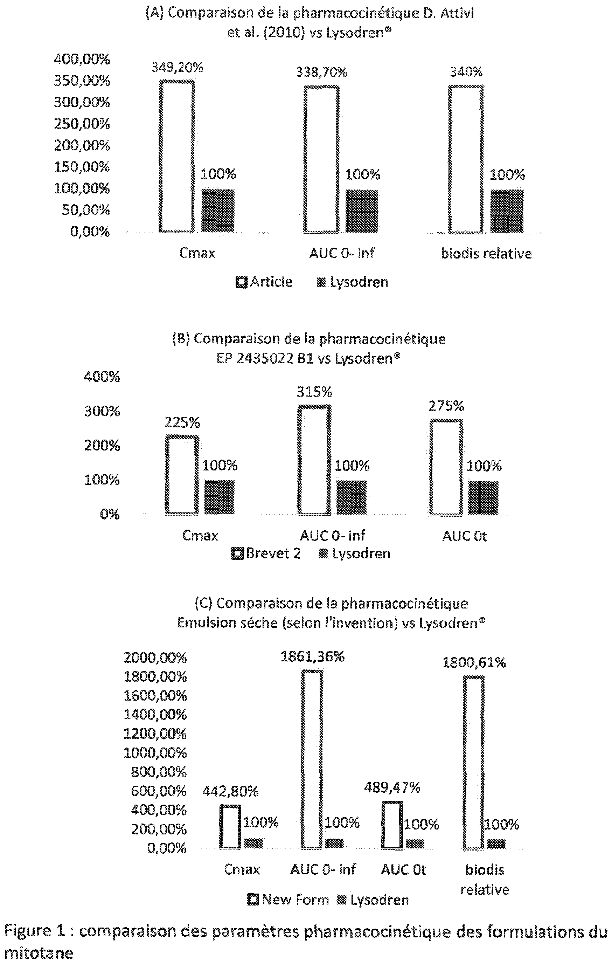

Les études de Pharmacocinétique chez le lapin ont montré une amélioration de la biodisponibilité, multipliée par un facteur 3 comparativement à celle de la spécialité Lysodren^{®} (Tableau 1) et [Fig 1A].

**[Table 1]**

| | | **Doses (100 mg/Kg) (n=3) (Lapin)** | |
|---|---|---|---|
| **Paramètres** | **unités** | **Lysodren ^{©}** | **SAE (Attivi et al.,2012)** |
| Cₘₐₓ | mg/L | 0,63 | **2,2** |
| Cₘₐₓ | H | 3,3 | **3,2** |
| AUC_{0-∞} | mg h/L | 3,1 | **10,5** |
| Biodisponibilité relative | | 1 | **3,4** |

### [Table 1] Paramètres pharmacocinétiques selon Attivi et al., (chez le lapin)

- La seconde, le brevet EP2435022 B1 concerne une invention qui décrit un SAE de mitotane sous forme de capsules molles qui comprend du mitotane dissous dans une matrice comprenant :
   - le monocaprylate de propylène glycol;
   - le dicaprate de propylène glycol
   - le monooléate de polyoxyéthylène sorbitanne Une étude de biodisponibilité menée chez le chien a également montré une amélioration multipliée par un **facteur 3** comparativement à celle de la spécialité Lysodren^{®} [Fig 1B]. Une amélioration de biodisponibilité multipliée par un **facteur 3** reste très faible pour réduire le délai de 3 mois d'attente thérapeutique et pour réduire le nombre de prises de médicament par jour.
- Et en dernier, le Brevet WO 2012/071043 A1**,** une invention qui décrit une autre formulation de mitotane basée sur le concept du SAE avec plus de 70 % de tensio-actifs et sous la forme de capsule molle.
Aucune étude de biodisponibilité n'a été effectuée.

Une autre étude décrit un complexe soluble à base de mitotane et cyclodextrine (Alfonsi R et al., Annales Pharmaceutiques Françaises. 2013; 71(3): 186-192.

Le défi est donc d'élaborer une nouvelle formulation de mitotane permettant d'obtenir une meilleure biodisponibilité comparativement au SAE afin de réduire le délai d'attente thérapeutique qui est sans doute le plus gros problème de mitotane, les variations inter et intra individuelles des taux plasmatiques ainsi que le nombre de prises de médicament par jour.

Le développement de systèmes lipidiques en une forme posologique solide (émulsion sèche (ES)) est une autre stratégie de formulation qui, outre l'amélioration de la biodisponibilité, offre d'autres avantages par rapport aux systèmes liquides ou semi solide. De tels systèmes impliquent la solidification des lipides liquides principalement en plusieurs unités telles que des poudres, des granulés, des comprimés, mini comprimés et des pellets à partir de cette poudre. Par conséquent, l'émulsion sèche (ES) combine les avantages des SAE liquides, comme par exemple une biodisponibilité accrue, une efficacité et une sécurité d'utilisation, avec ceux des formes posologiques solides (poudre), comme par exemple une manipulation et une administration faciles, une meilleure observance du patient, une stabilité et une reproductibilité élevées, une production plus rapide et plus facile à moindre coût. Plus précisément, ils offrent les avantages suivants:
- Ils réduisent le risque d'interactions des excipients du système lipidique avec l'enveloppe de la capsule, offrant ainsi une amélioration de la stabilité due à la réduction du risque de dégradation chimique et de croissance microbienne et impliquant une durée de conservation améliorée (Ma, H et al., Chem. Pharm. Taureau. 2014,62, 1173-1179).
- Ils peuvent être administrés sous forme de formulations à libération immédiate ou contrôlée en fonction du choix du ou des excipient(s) en poudre.
- La dose est présentée dans le poids précis de la poudre d'émulsion sèche, des granules, des mimi-comprimés ou des pellets remplis dans une gélule ou transformés en comprimés.
- Le coût de production est considérablement moindre comparé au remplissage de capsules molles puisque les émulsions sèches, les granules et les pellets ont une excellente fluidité, permettant un remplissage rapide et reproductible des gélules ou des matrices de compression avec des taux de production élevés.
- En particulier, les granulés, les mini comprimés ou pellets d'émulsion sèche, en tant que systèmes multiparticulaires, procurent des avantages thérapeutiques, caractéristiques de ces formes. Ils favorisent la réduction de la variation du temps de vidange gastrique, le passage en douceur dans l'intestin et le faible risque de libération d'une forte dose de substance active (effet dumping). Tous ces facteurs conduisent à la minimisation ou l'élimination de l'inter et l'intra variabilité des taux plasmatiques (Abuhelwa YA; et al., AAPS J. 2016, 18, 1322-1333).

Une émulsion sèche (ES) est une formulation solide, préparée par séchage d'une émulsion primaire liquide qui contient dans sa phase aqueuse un support solide et à partir de laquelle une émulsion à phase continue peut être reconstituée après réhydratation *in vitro (*Remon and Corveleyn, Int J Pharm, 1998, 166,65-74) ou *in vivo* lors d'une administration par voie orale *(*Remon and Corveleyn, Int J Pharm, 1998, 173, 149-155).

Selon la nature de la phase dispersée, il existe deux types d'ES: (i) huile dans eau (H/E), (ii) eau dans l'huile (E/H). Dans les ES de type H/E, les gouttelettes d'huile (leur taille peut varier d'une ou plusieurs dizaines de micromètres à une dizaine de nanomètres selon qu'il s'agisse d'une macro-, micro- ou nano-émulsion) sont dispersées dans une matrice hydrosoluble *(*Christensen et al.,Int J Pharm, 2001, 212, 195-202). Avant d'être séchées, les émulsions primaires sont préparées à partir:
- **d'une phase huileuse (10-20%, m/m):**
   - des triglycérides à chaînes moyennes (comme le Miglyol^{®} 812 *(*Ahmed and Aboul-Einien, Eur J Pharm Sci, 2007, 35, 219-225), le Phosal^{®}53, le Labrafac^{®}CC), des huiles végétales triglycérides à chaînes longues (l'huile de soja *(*Pedersen et al., Int J Pharm,1998, 171, 257-270), de maïs, de sésame *(*Ahmed and Aboul-Einien, Eur J Pharm Sci, 2007, 35, 219-225), des glycérides polyglycosylés insaturés (Labrafil^{®} M 1944 CS), du polyglycéryl-6-di-oléate (Plurol^{®} Oléique CC 497) *(*Christensen et al.,Int J Pharm, 2001, 212, 187-194);
- **d'un ou de plusieurs surfactants (2-4%, m/m):**
   - Caséinates de sodium *(*Dollo et al., Eur J Pharm Sci, 2003, 19, 273-280), copolymère à bloc de polyéthylène glycol/polypropylène glycol (Poloxamer^{®} 188) *(*Christensen et al.,Int J Pharm, 2001, 212, 187-194), monooléate de polyoxyéthylène de sorbitane (Tween^{®} 80) *(*Ahmed et al., Eur J Pharm Sci, 2008, 35, 219-225);
   - **d'une phase aqueuse (70-80%, m/m)** qui contient des polymères hydrophiles (amidons naturels *(*Hansen et al., Int J Pharm, 2005, 293, 203-211) ou modifiés *(*Christensen et al., Int J Pharm, 2001, 212, 187-194), méthylcellulose Methocel^{®} E15LV *(*Remon and Corveleyn, Int J Pharm, 1998, 166, 65-74) ou des sucres (5-30%, m/m) (les maltodextrines *(*Jang et al., Eur J Pharm Sci, 2006, 28,405-411), le lactose *(*Yin et al., J Control Release, 2009,140,86-94), le tréhalose, le mannitol *(*Hansen et al., Int J Pharm, 2004,287,55-66) et le saccharose *(*Christensen et al., Eur J Pharm Biopharm, 2002,53,147-153).

Des émulsions sèches sont obtenues également par élimination de l'eau libre d'une émulsion primaire huile dans eau, par séchage par atomisation *(*Zhang et al., Int J Pharm, 2011,414,186-192), par lyophilisation *(*Ahmed et al., Eur J Pharm Sci,2008,35,219-225*)* ou par évaporation de la phase aqueuse sous vide *(*Zhang et al., Int J Pharm, 2011,415, 293-300). Les procédés de séchage par atomisation et lyophilisation sont les plus utilisés. La poudre ainsi obtenue peut, soit être utilisée directement telle quelle pour remplir des gélules, soit subir des traitements, comme par exemple une étape de granulation humide en milieu non aqueux, un compactage ou encore une compression donnant lieu à des comprimés *(*Hansen et al., Int J Pharm, 2005,293,203-211). Lorsque l'émulsion est réhydratée, les gouttelettes d'huile sont libérées pour former à nouveau une émulsion huile dans eau.

La formulation des ES n'est cependant pas simple à mettre en œuvre; le séchage reste une étape très critique. La lyophilisation est un procédé long, complexe, exigeant et qui coûte cher (investissement et consommation énergétique). Les ES présentent donc certains inconvénients: les poudres obtenues après séchage des émulsions sont généralement volumineuses, cohésives, avec une mauvaise coulabilité, hygroscopique, ce qui les rend difficiles à manipuler sans traitement supplémentaire *(*Christensen et al., Int J Pharm, 2001,212, 195-202).

L'invention a pour objet la préparation et l'utilisation d'une nouvelle formulation du mitotane sous forme d'émulsion sèche (poudre) et son utilisation pour la voie orale.

L'inventeur a découvert de manière tout à fait surprenante un procédé de fabrication pour l'obtention d'une émulsion sèche contenant le mitotane en évitant l'élimination de la phase contenue liquide de l'émulsion primaire par adsorption, lyophilisation ou atomisation.

Les travaux des inventeurs ont montré qu'il était possible de formuler une nouvelle formulation de mitotane sous forme d'émulsion sèche (ESM), composée à base de substances huileuses, la phase huileuse est une huile végétale ou leurs mélanges, une huile animale ou leurs mélanges, et/ou une huile marine ou leurs mélanges est présente à une teneur inférieure à 50 % en poids et de cyclodextrines. Les Composition pharmaceutique et vétérinaire comprenant une formulation d'émulsion sèche de mitotane, cette nouvelle formulation de mitotane est utilisable telle quelle ou pour remplir des gélules, sachets ou sticks mais aussi, avec ou sans étape (s) de granulation, elle peut être comprimée pour donner lieu à des comprimés par compression, et permettant l'obtention des pellets par extrusion sphéronisation.

L'invention a donc pour but de fournir une nouvelle formulation de mitotane sous forme d'émulsion sèche (ESM), sous forme de poudre dispersible dans l'eau ou dans les milieux biologiques, sans tensioactifs et sans solvants organiques, et à base de cyclodextrines choisie parmi l'a-cyclodextrine, la β-cyclodextrine et la γ-cyclodextrine et en ce que les dérivés de cyclodextrine sont choisis parmi les dérivés hydroxypropylés, méthylés, éthylés, sulfobutylés éther ou acétylés de l'a-cyclodextrine, de la β-cyclodextrine et de la γ-cyclodextrine et les mélanges binaires ou ternaires desdites cyclodextrines et desdits dérivés de cyclodextrines, et est présente à une teneur supérieure à 45 % en poids pour éviter les phénomènes de recristallisation et de précipitation du mitotane.

Les émulsions sèches de mitotane (ESM) selon l'invention sont caractérisées en ce qu'elles sont élaborées à partir de substances huileuses chargées de mitotane avec ou sans cosolvant ainsi avec ou sans promoteurs d'absorption qui sont choisis par exemple parmi les composés suivants: glycéryl caprylate/caprate, Macrogolglycerol hydroxystearate, Macrogolglycerol ricinoleate (Cremophor EL^{®}), oléate de sorbitane polyoxyéthylène, éther monoéthylique du diéthylène glycol, monocaprylate de propylène glycol, éthanol absolu, et macrogol 800 à 300, et qui sont présent à une teneur de 10 à 20 % en poids_et de cyclodextrines ou leur mélange, et forment après ajout d'une phase aqueuse une émulsion primaire eau dans huile (E/H), un ensemble essentiellement solide sous forme de poudre.

Procédé de préparation de l'émulsion sèche de mitotane approprié pour former un système d'administration de mitotane, lequel comprend la solubilisation du mitotane dans une phase huileuse avec ou sans co-solvant, l' ajout de cyclodextrine dans la phase huileuse avec ou sans promoteur d'absorption, l'ajout de la phase aqueuse pour l'obtention d'une émulsion primaire H/E puis d'une émulsion sèche à base de mitotane, un Séchage et un calibrage des grains contenant des proportions d'huile, de cyclodextrine et de mitotane inférieures à 50%, inférieures à 60% et supérieures à 6 % respectivement

### Exemple 1 : Préparation d'une émulsion sèche E/H contenant le mitotane (ESM)

Au cours d'une seule étape, on introduit dans un mélangeur planétaire (type Hobart) 25 ml de mélange d'huile de maïs chargés de 5 grammes de mitotane et de 3 ml d'éthanol en tant que co-solvant pour améliorer la solubilité du mitotane dans la phase huileuse. On ajoute sous agitation (variateur N°1) et à température ambiante (25°C) 42.5 grammes d'a-cyclodextrines dispersés dans la phase huileuse. L'émulsion sèche de mitotane E/H est formée après ajout d'une phase aqueuse (5 ml d'eau) sous agitation (variateur N°2). Les granules humides sont alors calibrées (maille de 1 µm) dans un granulateur oscillant, puis séchées dans une étuve à 45°C pendant 15 minutes jusqu'à une teneur en humidité de 5 à 6% et élimination d'alcool. On obtient des granules de taille moyenne de 800 µm chargées de 6.8% de mitotane

### Exemple 2 : Préparation d'une émulsion sèche E/H contenant le mitotane (ESM) avec promoteur d'absorption

On opère comme décrit dans l'exemple 1, mais en utilisant 10 à 20% de cremophore EL^{®} ou polysorbate 80 comme promoteur d'absorption du mitotane. On obtient des granules de taille moyenne de 800 µm chargées de 6.8% de mitotane.

### Exemple 3 : Préparation et évaluation biopharmaceutique d'une émulsion sèche E/H contenant le mitotane (ESM)

### - ETUDE PRECLINIQUE

L'administration par voie orale de mitotane sous forme d'émulsion sèche (ESM) préparée selon **l'exemple 1,** a été évaluée chez six rats (Charles River^{®}) de poids moyen de 350 g. La posologie administrée par voie orale est de 100 mg / kg pour la formule innovante et a été comparée à la spécialité Lysodren^{®} (médicament de référence commercialisé). Les prélèvements ont été réalisés à des temps réguliers : 0, 1H, 2H, 3H, 4H, 5H, 7H et 9H. Le dosage plasmatique de mitotane est réalisé à partir d'un échantillon de sang prélevé sur tube sec, hépariné. Ces prélèvements sont centrifugés et le surnageant est conservé dans un tube à hémolyse en verre à une température de -20 °C. Le plasma est purifié par précipitation avec du méthanol. L'analyse chromatographique (CLHP) utilise une colonne LiChrospher 100^{®} C₈ de 5 µm de diamètre et thermostatée à 40 °C, avec une phase mobile isocratique, composée d'un mélange acétonitrile et tampon acétique pH 3,2 (75:25). Le débit est de 1,2 mL/min. Le mitotane est détectés à une longueur d'onde de 234 nm. Le temps de rétention est à 6 min pour une durée d'analyse d'échantillon de 9 min.

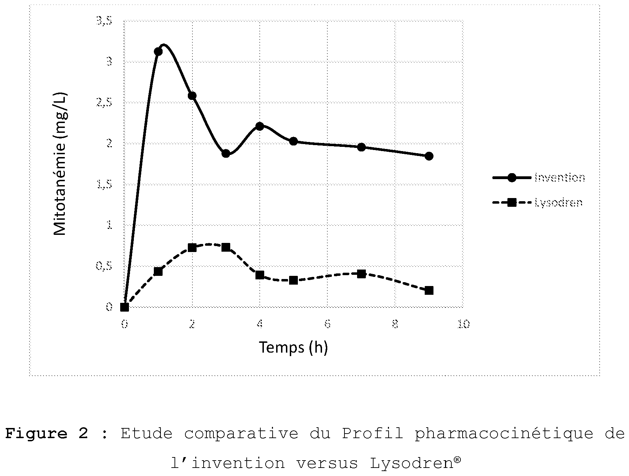

Les concentrations plasmatiques de mitotane sous forme d'émulsion sèche (ESM) (selon l'invention), comparées à celles de la spécialité de référence Lysodren^{®}, sont représentées dans le tableau 2 et [Fig 2].

**[Table 2]**

| | | **Doses (100 mg/Kg) (n=3) (Rats)** | |
|---|---|---|---|
| **Paramètres** | **unités** | **Lysodren ^{©}** | **Emulsion sèche (invention)** |
| Cₘₐₓ | mg/L | 0,7 | **3,1** |
| Tₘₐₓ | H | 2 | **1** |
| t_{1/2} | H | 1,930 | **23,866** |
| AUC_{0-∞} | mg h/L | **4,4** | **81,9** |
| AUMC_{∞} | mg h^{x}h/L | 22,2 | **2834,4** |
| MRT_{0-∞} | H | 5,1 | **34,6** |
| Vd | L | 63,9 | **42** |
| CL | L/H | 21,768 | **1,176** |
| t_{1/2} (de Vd & CL) | H | 1,9 | **23,9** |
| Biodisponibilité relative | | 1 | **18,61** |

### [Table 2] Paramètres pharmacocinétiques de l'invention (chez le rat)

Les principaux résultats obtenus sont les suivants :
- L'aire sous la courbe (AUC) a été multipliée par facteur **28** par rapport à celle de Lysodren^{®}
- Le temps maximum (Tₘₐₓ) a été **réduit de moitié** par rapport à celui de Lysodren^{®}.
   Une analyse des résultats entre l'émulsion sèche de mitotane (ESM), les travaux publiés selon D. Attivi (Attivi et al., Drug Dev Ind Pharm. 2010 Apr;36(4):421-7). et le brevet EP2435022 B1 comparativement à la spécialité Lysodren^{®} est présentée dans la [Fig 1] :
- Concernant **l'AUC 0-inf:** le système autoémulsionnant (SAE) de mitotane développé par Attivi (Attivi et al., Drug Dev Ind Pharm. 2010 Apr;36(4) :421-7) montre une amélioration multipliée par un facteur de près de **3.4,** le brevet EP 2435022 permet l'amélioration d'un facteur **3.15** et la présente invention permet l'obtention d'une AUC 0-inf multipliée par un facteur de près de **19.**
- Concernant **l'AUC 0t:** le système autoémulsionnant (SAE) de mitotane développé selon le brevet EP 2435022 montre une amélioration multipliée par un facteur de **2.75** et la présente invention permet l'obtention d'une AUC 0-inf multipliée par un facteur de près de **4.9.**
- Concernant **la biodisponibilité relative:** le système autoémulsionnant (SAE) de mitotane développé par Attivi *et al.,* montre une amélioration multipliée par un facteur **3.4** et la présente invention permet l'obtention d'une biodisponibilité relative multipliée par un facteur **18.**
- Concernant la **Cmax:** le système autoémulsionnant (SAE) de mitotane développé par Attivi *et al.,* montre une amélioration multipliée par un facteur **3.5,** le brevet EP 2435022 permet l'amélioration d'un facteur **2.2** et la présente invention permet l'obtention d'une Cmax multipliée par un facteur de près de **4.5.**

## Revendications

1. Formulation d'émulsion sèche de mitotane comprenant :
Une phase huileuse enrichie de mitotane et de cyclodextrine contenant ou non un co-solvant et/ou un promoteur d'absorption.

2. Formulation selon la revendication 1, dans laquelle la phase huileuse est une huile végétale ou leurs mélanges, une huile animale ou leurs mélanges, et/ou une huile marine ou leurs mélanges est présente à une teneur inférieure à 50 % en poids.

3. Formulation selon la revendication 1, dans laquelle la phase huileuse comprennent des promoteurs d'absorption et/ou des co-solvants qui sont choisis par exemple parmi les composés suivants: glycéryl caprylate/caprate, Macrogolglycerol hydroxystearate, Macrogolglycerol ricinoleate (Cremophor EL^{®}), oléate de sorbitane polyoxyéthylène, éther monoéthylique du diéthylène glycol, monocaprylate de propylène glycol, éthanol absolu, et macrogol 800 à 300, et qui sont présent à une teneur de 10 à 20 % en poids.

4. Formulation selon la revendication 1, selon laquelle la cyclodextrine est choisie parmi l'a-cyclodextrine, la β-cyclodextrine et la γ-cyclodextrine et en ce que les dérivés de cyclodextrine sont choisis parmi les dérivés hydroxypropylés, méthylés, éthylés, sulfobutylés éther ou acétylés de l'a-cyclodextrine, de la β-cyclodextrine et de la γ-cyclodextrine et les mélanges binaires ou ternaires desdites cyclodextrines et desdits dérivés de cyclodextrines, et est présente à une teneur supérieure à 45 % en poids.

5. Composition pharmaceutique et vétérinaire comprenant une formulation d'émulsion sèche de mitotane telle que définie dans l'une des revendications 1 à 4.

6. Composition selon la revendication 5, se présentant sous une forme appropriée pour une administration par voie orale.

7. Composition selon la revendication 6, telle qu'elle se présente sous différentes formes pelliculées ou non :
d'une poudre, de granulés, de mini-comprimés, de granules (pellets), de comprimés, de gélules.

8. Formulation se présentant sous la forme de capsules molles ou capsules dures contenant du mitotane dans une phase huile et la cyclodextrine, telle que définie dans l'une quelconque des revendications 2 à 4.

9. Composition selon l'une quelconque des revendications 6 à 7, pour une utilisation dans le traitement du carcinome corticosurrénalien, de l'hyperplasie surrénale congénitale et du syndrome de Cushing.

10. Procédé de préparation de l'émulsion sèche de mitotane selon les revendications 2 à 4 pour former un système d'administration de mitotane, lequel comprend la solubilisation du mitotane dans une phase huileuse avec ou sans co-solvant, l'ajout de cyclodextrine dans la phase huileuse avec ou sans promoteur d'absorption, l'ajout de la phase aqueuse pour l'obtention d'une émulsion primaire H/E puis d'une émulsion sèche à base de mitotane, un Séchage et un calibrage des grains.

## Patentansprüche

1. Trockenemulsionsformulierung von Mitotan, enthaltend:
eine mit Mitotan und Cyclodextrin angereicherte Ölphase, die ein Co-Lösungsmittel und/oder einen Absorptionspromotor enthält oder nicht.

2. Formulierung nach Anspruch 1, bei der die Ölphase ein Pflanzenöl oder Mischungen davon, ein tierisches Öl oder Mischungen davon und/oder ein marines Öl oder Mischungen davon in einem Anteil von weniger als 50 Gew.% vorliegt.

3. Formulierung nach Anspruch 1, wobei die Ölphase Absorptionspromotoren und/oder Co-Lösungsmittel umfasst, die beispielsweise aus den folgenden Verbindungen ausgewählt sind: Glycerylcaprylat/Caprat, Macrogolglycerolhydroxystearat, Macrogolglycerolricinoleat (Cremophor EL^{®}), Polyoxyethylensorbitanoleat, Diethylenglykolmonoethylether, Propylenglykolmonocaprylat, absolutes Ethanol und Macrogol 800 bis 300, und die in einem Anteil von 10 bis 20 Gew.% vorliegen.

4. Formulierung nach Anspruch 1, bei der das Cyclodextrin unter 1'a-Cyclodextrin, b-Cyclodextrin und g-Cyclodextrin ausgewählt ist, und die Cyclodextrin-Derivate unter hydroxypropylierten, methylierten, ethylierten, sulfobutylierten Ether- oder Acetylderivaten oder acetyliertem α-Cyclodextrin, β-Cyclodextrin und γ-Cyclodextrin und binären oder ternären Mischungen dieser Cyclodextrine und dieser Cyclodextrinderivate ausgewählt sind, und es in einem Anteil von mehr als 45 Gew.% vorliegt.

5. Pharmazeutische und veterinärmedizinische Zusammensetzung, enthaltend eine Trockenemulsionsformulierung von Mitotan, wie in einem der Ansprüche 1 bis 4 definiert.

6. Zusammensetzung nach Anspruch 5, die in einer zur oralen Verabreichung geeigneten Form vorliegt.

7. Zusammensetzung nach Anspruch 6, wie sie in verschiedenen filmbeschichteten oder unbeschichteten Formen vorliegt: Pulver, Granulat, Minitabletten, Granulat (Pellets), Tabletten, Kapseln.

8. Formulierung in Form von Weichkapseln oder Hartkapseln, die Mitotan in einer Ölphase und Cyclodextrin enthalten, wie in einem der Ansprüche 2 bis 4 definiert.

9. Zusammensetzung nach einem der Ansprüche 6 bis 7 zur Verwendung bei der Behandlung von Nebennierenrindenkarzinomen, angeborener Nebennierenhyperplasie und Cushing-Syndrom.

10. Verfahren zur Herstellung der Trockenemulsion von Mitotan gemäß den Ansprüchen 2 bis 4 zur Bildung eines Systems für die Verabreichung von Mitotan, das die Solubilisierung von Mitotan in einer Ölphase mit oder ohne Co-Lösungsmittel, die Zugabe von Cyclodextrin in die Ölphase mit oder ohne Absorptionspromotor, die Zugabe der wässrigen Phase, um eine O/W-Primäremulsion und dann eine Trockenemulsion auf Mitotan-Basis zu erhalten, Trocknung und Sortieren der Körner umfasst.

## Claims

1. A formulation of mitotane dry emulsion comprising:
An oily phase enriched with mitotane and cyclodextrin containing, or not, a co-solvent and/or an absorption promoter.

2. The formulation according to claim 1, wherein the oily phase is a vegetable oil or mixtures thereof, an animal oil or mixtures thereof, and/or a marine oil or mixtures thereof is present at a content lower than 50 weight%.

3. The formulation according to claim 1, wherein the oily phase comprises absorption promoters and/or co-solvents which are selected, for example, from the following compounds: glyceryl caprylate/caprate, Macrogolglycerol hydroxystearate, Macrogolglycerol ricinoleate (Cremophor EL^{®}), polyoxyethylene sorbitan oleate, diethylene glycol monoethyl ether, propylene glycol monocaprylate, absolute ethanol, and macrogol 800 to 300, and which are present at a content of 10 to 20 weight%.

4. The formulation according to claim 1, according to which the cyclodextrin is selected from 1'α-cyclodextrin, β-cyclodextrin and γ-cyclodextrin and in that the cyclodextrin derivatives are selected from hydroxypropylated, methylated, ethylated, sulfobutylated ether derivatives or acetylated α-cyclodextrin, β-cyclodextrin and γ-cyclodextrin and binary or ternary mixtures of said cyclodextrins and of said cyclodextrin derivatives, and is present at a content higher than 45 weight%.

5. A pharmaceutical and veterinary composition comprising a dry emulsion formulation of mitotane as defined in any of claims 1 to 4.

6. The composition according to claim 5, in a form suitable for oral administration.

7. The composition according to claim 6, as it is in different film-coated or uncoated forms: a powder, granulates, mini-tablets, granules (pellets), tablets, capsules.

8. A formulation in the form of soft capsules or hard capsules containing mitotane in an oil phase and cyclodextrin, as defined in any one of claims 2 to 4.

9. The composition according to any one of claims 6 to 7, for use in the treatment of adrenocortical carcinoma, congenital adrenal hyperplasia and Cushing's syndrome.

10. A method for the preparation of the dry emulsion of mitotane according to claims 2 to 4 to form a system for administering mitotane, which comprises the solubilization of mitotane in an oily phase with or without a co-solvent, the addition of cyclodextrin in the oily phase with or without absorption promoter, the addition of the aqueous phase to obtain an O/W primary emulsion then a dry emulsion based on mitotane, drying and calibration of the grains.
